# FASCICULE DE BREVET EUROPEEN

(11) **EP 3 074 142 B1**
(45) Date de publication et mention de la délivrance du brevet: **12.12.2018**
(21) Numéro de dépôt: 14800110.0
(22) Date de dépôt: 13.10.2014
(51) Int. Cl.: B05B 11/00, A61M 15/08, A61M 11/00, B65D 83/54

(54) **POMPE AVEC REPRISE D'AIR**
ATMOSPHÄRISCHE PUMPE
PUMP OF THE ATMOSPHERIC TYPE

(30) Priorité: 26.11.2013 FR 1361626
(43) Date de publication de la demande: 05.10.2016
(73) Titulaire: Nemera La Verpilliere, F-38290 La Verpilliere (FR)
(72) Inventeur: DONNETTE, Xavier, 38510 Courtenay (FR); CAMBA, José, 01500 Amberieu en Bugey (FR); SAHM, Philippe, 73100 Aix Les Bains (FR); MONGIN, Xavier, 38360 Sassenage (FR); GENIN, Jérôme, 69003 Lyon (FR)
(74) Mandataire: de la Bigne, Guillaume Michel Marie
(86) Numéro de dépôt international: PCT/FR2014/052594
(87) Numéro de publication internationale: WO 2015/079132

(56) Documents cités:
- WO-A1-93/22360
- WO-A1-98/48943
- WO-A1-2012/150409
- FR-A1- 2 781 767
- FR-A1- 2 794 727
- FR-A1- 2 798 368
- FR-A1- 2 810 301
- FR-A1- 2 952 040
- FR-A1- 2 989 290

## Description

La présente invention concerne le domaine technique de la délivrance de produit liquide, semi-liquide ou visqueux notamment dans le domaine médical, plus particulièrement un dispositif pour la délivrance d'un produit par pulvérisation. Ce dispositif comprend plus précisément une pompe, permettant d'aspirer et distribuer du produit contenu dans un réservoir. Le dispositif peut être utilisé en particulier pour des pulvérisations nasales.

Selon un exemple de pompe décrit dans le document FR 2 885 890, la pompe comprend une membrane élastique comprenant une partie formant une valve anti-retour de façon à empêcher le reflux de produit depuis une chambre de dosage vers un réservoir. La membrane élastique est rapportée sur un support traversé par un canal d'alimentation, servant de siège à la partie formant valve anti-retour de sorte que la valve anti-retour soit plaquée contre le siège pour bloquer le liquide ou distante du siège pour le laisser passer. La membrane est montée coulissante dans la chambre de dosage de la pompe, de façon étanche, pour expulser hors de la chambre du produit contenu dans la chambre de dosage.

On sait que certaines pompes, telles que celle-ci, sont configurées pour délivrer du produit sans reprise d'air, ce qui permet notamment d'éviter d'utiliser des conservateurs pour assurer la stérilité du produit. Ainsi, à chaque activation de la pompe, une dépression se crée dans le réservoir.

Il se trouve que, au cours de l'utilisation, lorsque la dépression dans le réservoir devient importante, la dose de produit délivré peut ne pas être assez précise.

On connaît certes des pompes avec reprise d'air, permettant de faire entrer de l'air dans le réservoir pour combler la dépression. Néanmoins, l'air entrant peut introduire des bactéries nuisibles à la stérilité du produit, même lorsqu'un filtre poreux est utilisé pour filtrer l'air.

Le document WO98/48943 décrit un distributeur de produit liquide comportant un moyen d'obturation contrôlée de l'évent.

L'invention a pour but de remédier notamment à cet inconvénient en fournissant une pompe pour la délivrance d'un produit permettant de délivrer des doses précises tout en assurant la stérilité du produit contenu dans le réservoir.

L'invention a pour objet un dispositif pour la délivrance d'un produit par pulvérisation, comportant :
- un réservoir pour le stockage du liquide à distribuer, et
- une pompe comportant un corps de pompe monté fixe sur le réservoir, le corps de pompe comportant un orifice de passage d'air depuis l'extérieur vers l'intérieur du réservoir,
- le dispositif comportant un passage d'air depuis l'extérieur vers l'intérieur du réservoir, traversant l'orifice de passage d'air, le passage d'air étant obturé par un organe réalisé en matériau polymère perméable à l'air, ce matériau étant non poreux, l'organe étant appelé organe perméable à l'air ;
l'organe perméable à l'air est fixé par pincement entre deux éléments du dispositif rapportés l'un sur l'autre.

On propose donc de réaliser une pompe avec reprise d'air dans le réservoir, ce qui permet de délivrer des doses plus précises et plus reproductibles. En effet, lorsque la dépression dans le réservoir devient importante, le fonctionnement de la pompe devient plus difficile car la membrane élastique formant valve anti-retour tend à rester plaquée contre son siège. Ainsi, l'aspiration nécessaire pour éloigner la membrane de son siège est de plus en plus importante.

Les doses administrées sont également plus précises car on évite la formation de bulles d'air dans la chambre de dose provoquées par un phénomène d'équilibrage de pression dû à l'augmentation des différences de pression de part et d'autre de la membrane.

De plus, la reprise d'air non contaminé permet également de délivrer tout le produit contenu dans le réservoir. En effet, lorsque l'aspiration de la pompe n'est plus suffisante pour contrer la dépression à l'intérieur du réservoir, la pompe peut se bloquer et ce, alors que le réservoir contient encore du produit.

En outre, la fonction de reprise d'air dans le réservoir est assurée, non pas en procédant à une filtration de l'air, mais en utilisant les propriétés de diffusion de gaz de certains matériaux. Ainsi, on utilise un autre type d'organe qu'un filtre, à savoir un organe en matériau polymère non poreux. Un tel organe présente l'avantage d'assurer le passage d'air non contaminé de façon plus fiable qu'un filtre, lequel est poreux par définition.

En outre, avec un organe non poreux, il n'est pas nécessaire de tester la taille de pores et il est plus facile de tester qu'il n'y a pas de fuite due à un mauvais assemblage ou à un organe défectueux.

Par ailleurs, comme l'orifice de passage d'air est généralement de taille très réduite, le positionnement du filtre doit être particulièrement précis sur le corps de pompe et implique donc un procédé de fabrication et d'assemblage coûteux. Grâce à l'utilisation d'un matériau non poreux, dont les coûts de fabrication peuvent être moins élevés que ceux d'un filtre antibactérien, on peut prévoir un organe perméable à l'air présentant une surface suffisamment grande pour faciliter son positionnement sur le dispositif.

Par matériau « non poreux », on entend une matière pleine, sans trou, bloquant le passage de particules telles que des bactéries, par exemple la bactérie Brevundimonas Diminuta ayant une taille de l'ordre de 0,2 micromètres. Ce matériau non poreux se distingue d'un filtre, réalisé de façon à être poreux. En effet, le matériau non poreux proposé pour l'organe perméable à l'air est composé d'un polymère utilisé dans sa forme brute, ayant subi par exemple une simple injection ou compression, alors qu'un matériau poreux tel que celui d'un filtre est composé d'un polymère ayant subi en outre des étapes de génération de pores ou interstices, telles qu'un étirement sur la matière ou une addition de solvant chimique dans le polymère. Le matériau étant non poreux, il est étanche aux liquides et ne laisse pas passer de particules telles que des poussières ou des microorganismes. En revanche, ce matériau est perméable à l'air, du fait qu'il laisse passer des éléments de la taille d'une molécule. En d'autres termes, le matériau non poreux proposé ci-dessus a une perméabilité aux gaz qui laisse passer les molécules d'air, à travers un réseau réticulé de longues chaînes moléculaires enchevêtrées. On notera que, du fait que le matériau est non poreux, le passage d'air à travers l'organe perméable à l'air est un processus qui prend plusieurs minutes, voire plusieurs heures, et non quelques secondes, comme c'est le cas pour un filtre. Ainsi, il est plus facile de vérifier par des tests de courtes durées l'absence de fuite du dispositif.

Lorsque la pompe revient à une position de repos, la dépression entre l'intérieur et l'extérieur du flacon est compensée, lentement, par le passage d'air extérieur à travers l'organe perméable à l'air.

On notera que, comme cet organe perméable à l'air ne comporte pas de pores, il n'y a pas de risque de colmatage dû à une accumulation de microorganismes et de poussières dans les pores. Par ailleurs, il n'y a pas non plus de pression capillaire de liquide, lequel s'oppose au rétablissement de l'équilibre des pressions entre l'intérieur et l'extérieur du réservoir lorsque le dispositif est en position « tête en bas » et que du liquide est alors en contact avec l'organe perméable à l'air. Ces deux phénomènes sont présents lorsque l'on utilise un filtre.

Comme l'organe perméable à l'air est fixé par pincement entre deux éléments du dispositif rapportés l'un sur l'autre, il est simple et facile à mettre en oeuvre.

Le dispositif peut en outre comporter l'une ou plusieurs des caractéristiques suivantes, prises seules ou en combinaison.
- Le corps de pompe et l'organe perméable à l'air ont chacun un axe central, les deux axes étant confondus. Cela permet de faciliter les opérations de montage de l'organe sur le corps de pompe. En effet, il est facile de centrer une pièce par rapport à une autre. En outre, comme l'organe perméable à l'air présente un axe central, il peut avoir une forme de révolution, capable d'entourer une partie du dispositif, donc de couvrir un passage d'air étendu. L'organe perméable à l'air a de préférence une forme générale de manchon. On peut prévoir ainsi qu'il ait une surface relativement grande, par exemple couvrant tout le col du réservoir du dispositif, et puisse ainsi offrir une plus grande surface de passage d'air, pour que l'équilibre des pressions interne et externe se réalise au plus vite.
- L'organe perméable à l'air est élastiquement déformable et fixé par auto-serrage mécanique élastique autour d'au moins un élément du dispositif. Grâce à l'élasticité de l'organe perméable à l'air, ce dernier peut se déformer et venir serrer l'élément du dispositif autour duquel il est positionné. Ce mode de fixation est simple et facile à mettre en oeuvre.
- L'organe perméable à l'air comporte une paroi dite de passage d'air, munie d'une pluralité de reliefs, pour augmenter la surface de passage d'air. Par exemple, on peut prévoir une paroi ondulée, à section sinusoïdale, en accordéon, en créneaux ou encore en zig-zag. Ainsi, on augmente la surface d'échange d'air entre l'intérieur et l'extérieur du réservoir sans pour autant augmenter considérablement l'encombrement de l'organe perméable à l'air. En effet, le flux d'air pouvant passer à travers la paroi de l'organe perméable à l'air par perméabilité est directement proportionnel à la surface d'échange et inversement proportionnel à l'épaisseur de la paroi de l'organe perméable à l'air. Une grande surface d'échange et une épaisseur faible de paroi favorisent la reprise d'air. Selon la vitesse de reprise d'air souhaitée, on pourra donc facilement modifier la géométrie de la paroi de l'organe perméable à l'air en faisant varier les paramètres de surface d'échange et d'épaisseur. On comprend que les reliefs ménagés sur la paroi se distinguent de nervures, ils sont réalisés en parallèle sur les deux faces de la paroi de l'organe perméable à l'air, l'épaisseur de la paroi étant sensiblement constante le long des reliefs et suffisamment faible pour autoriser le passage d'air, de façon à augmenter la surface d'échange de l'organe perméable à l'air. En outre, ces reliefs permettent une déformation plus aisée de l'organe perméable à l'air.

- La pompe peut prendre une position de repos et une position activée, le passage d'air étant fermé lorsque la pompe est en position de repos. Le passage d'air est fermé de façon étanche à l'air. Ainsi, on limite les risques d'évaporation du produit contenu dans le réservoir lorsque la pompe n'est pas utilisée, notamment pendant le stockage de la pompe avant son utilisation. Par exemple, la pompe présente une partie fixe et une partie mobile, montée mobile par rapport à la partie fixe, cette partie mobile présentant une surface en contact étanche avec la partie fixe lorsque la pompe est en position de repos et le contact étanche étant rompu lorsque la pompe est en position activée, le passage d'air étant alors ouvert.
- L'organe perméable à l'air est rapporté autour du corps de pompe. L'organe perméable à l'air étant directement rapporté autour du corps de pompe, son positionnement dans le dispositif est simple.
- L'organe perméable à l'air comporte des nervures de renfort ménageant un espace libre entre une surface intérieure de l'organe perméable à l'air et le corps de pompe. Ces nervures de renfort permettent de rigidifier l'organe perméable à l'air et de ménager au moins un espace libre, entre la paroi de l'organe perméable à l'air et le corps de pompe, dans lequel l'air peut circuler ou être stocké temporairement avant de passer par diffusion, à travers de l'organe perméable à l'air, dans le réservoir. On comprend que de telles nervures correspondent à des augmentations locales de l'épaisseur de la paroi de l'organe perméable à l'air, notamment pour la rigidifier, formant ainsi des saillies sur une seule ou sur les deux faces de la paroi. Ces nervures se distinguent donc de reliefs qui auraient pour fonction d'augmenter la surface d'échange.
- L'organe perméable à l'air comprend en outre, une portion radiale formant au moins en partie joint d'étanchéité entre le réservoir et le corps de pompe, la portion radiale étant fixée, par exemple par pincement, entre le réservoir et le corps de pompe. L'organe perméable à l'air remplit donc la fonction de reprise d'air non contaminé et assure, au moins en partie, l'étanchéité du dispositif entre le corps de pompe et le réservoir de sorte que le produit contenu dans le réservoir ne peut pas en sortir et que l'air extérieur contaminé ne peut y entrer, entre le réservoir et le corps de pompe.
- La portion radiale de l'organe perméable à l'air est séparée du réservoir ou du corps de pompe par un joint formant un bourrelet enveloppant radialement la portion radiale de l'organe perméable à l'air et formant au moins en partie joint d'étanchéité entre le réservoir et le corps de pompe. Grâce au joint format bourrelet, l'organe perméable à l'air n'est pas en permanence en contact avec l'air à l'extérieur du dispositif. On assure ainsi une meilleure étanchéité entre le réservoir et le corps de pompe en minimisant les risques d'évaporation du produit, sous forme de molécules de vapeur d'eau par exemple, contenu dans le réservoir par diffusion à travers la portion radiale de l'organe perméable à l'air, notamment pendant la période de stockage du dispositif qui peut durer plus d'une année. Cette diffusion se ferait au détriment de la précision de la dose de principe actif délivrée. En effet, si du liquide s'évapore, le principe actif restant dans le réservoir est plus concentré et la dose de principe actif délivré lors de chaque activation du dispositif est plus importante que celle initialement prévue.
- La pompe présente une partie fixe et une partie mobile, montée mobile par rapport à la partie fixe, l'organe perméable à l'air entourant au moins partiellement la partie fixe et la partie mobile et étant apte à se déformer élastiquement lors du déplacement de la partie mobile.
- La partie fixe entourée par l'organe perméable à l'air comprend un organe de fixation de la pompe sur le réservoir.
- La pompe présente une partie fixe et une partie mobile, montée mobile par rapport à la partie fixe, l'organe perméable à l'air entourant au moins partiellement le réservoir et la partie mobile et étant apte à se déformer élastiquement lors du déplacement de la partie mobile.
- Le matériau polymère comprend un matériau élastomère. L'organe perméable à l'air étant déformable, il peut éventuellement être monté sur le dispositif par déformation légère de l'organe perméable à l'air. Une fois mis en place, l'organe perméable à l'air peut reprendre sa forme initiale et être fixé par auto-serrage mécanique élastique sur une ou plusieurs parties du dispositif, ce qui simplifie sa mise en place. De plus, la souplesse de l'élastomère permet d'éviter plus facilement des jeux entre l'organe perméable à l'air et les parties sur lesquelles il vient en serrage mécanique, par adaptation des surfaces de contact de l'organe perméable à l'air avec les parois du dispositif.
- Le matériau polymère comprend du silicone (également appelé polysiloxane, composé inorganique formé d'une chaîne silicium-oxygène). La perméabilité aux gaz du silicone permet de favoriser davantage le processus de reprise d'air et d'en réduire le temps. De plus, le silicone présente l'avantage d'être inerte par rapport à une large gamme de produits pharmaceutiques.

Nous allons maintenant présenter des modes de réalisation de l'invention en référence aux dessins annexés sur lesquels :
- la figure 1 est une vue en coupe d'un dispositif en position de repos, selon un premier mode de réalisation ;
- la figure 2 est une vue en coupe du dispositif de la figure 1 en position activée ;
- la partie gauche de la figure 3 présente un agrandissement partiel du dispositif de la figure 1 et la partie droite de la figure 3 présente un agrandissement partiel du dispositif de la figure 1 avant fixation de la pompe sur le réservoir;
- la figure 4 est une vue en coupe de l'organe perméable à l'air de la figure 1 ;
- la figure 5 est une vue en coupe d'un deuxième mode de réalisation du dispositif ;
- les figures 6 et 7 sont des vues en coupe d'un dispositif selon un troisième mode de réalisation, respectivement en position de repos et en position activée ;
- les figures 8 à 9 sont des vues en coupe d'un dispositif en position de repos selon des quatrième et cinquième modes de réalisation ;
- les figures 10 à 11 sont des vues en coupe d'un dispositif en position de repos selon deux modes de réalisation non revendiqués.

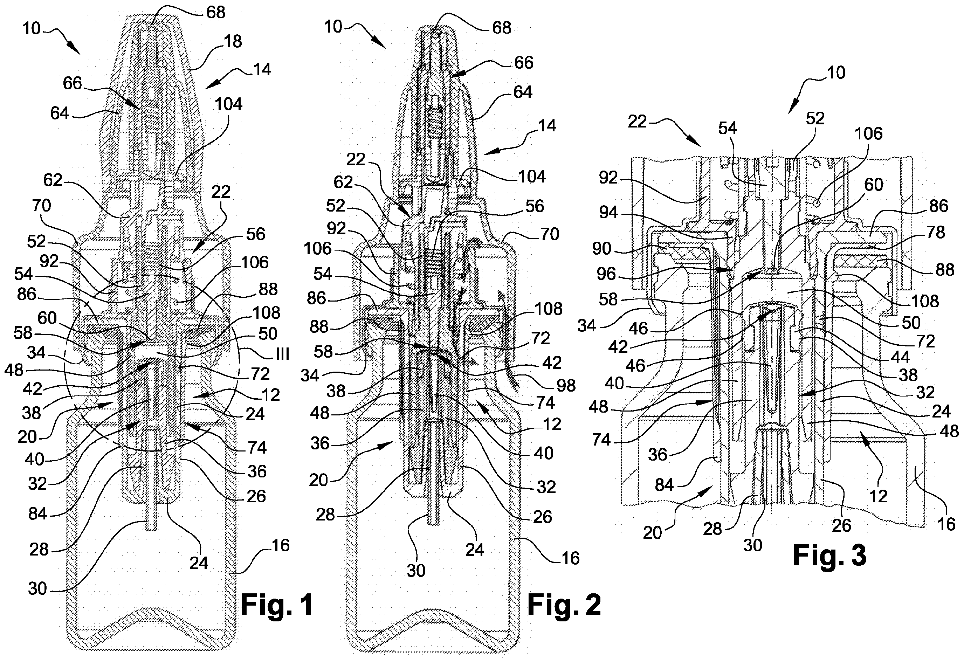

On a représenté sur les figures 1, 2 et 3 un dispositif 10 pour la délivrance d'un produit par pulvérisation comprenant une pompe 12 surmontée d'un embout de distribution 14 de produit. La pompe 12 est montée sur un réservoir 16 et l'embout de distribution 14 est couvert par un capot de protection 18 amovible. Le dispositif 10 est utilisé par exemple pour des pulvérisations nasales de produit pharmaceutique. Sur la figure 1, le dispositif 10 est présenté en position de repos, c'est-à-dire lorsqu'il n'est pas utilisé ou lorsqu'il est stocké.

La pompe 12 comprend une première partie 20, dite partie fixe, et une seconde partie 22, dite partie mobile, montée mobile par rapport à la partie fixe 20.

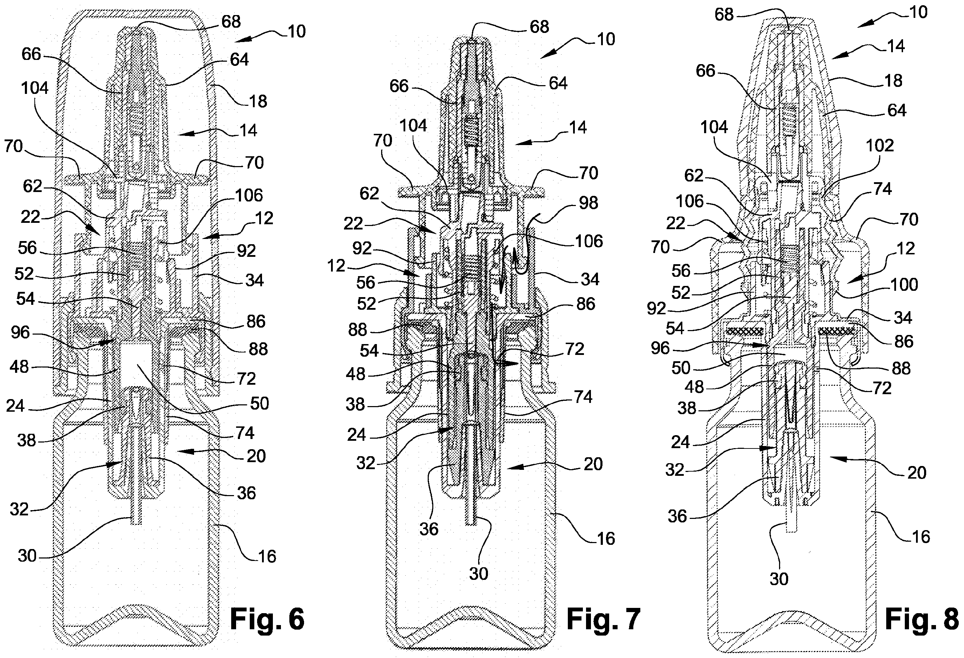

La partie fixe 20 de la pompe 12 comporte un corps de pompe 24 comprenant un cylindre extérieur 26 raccordé à un cylindre intérieur 28 de réception d'un tube plongeur 30. Le tube plongeur 30 est immergé dans le réservoir 16 lorsque la pompe 12 est montée dessus afin de puiser du produit à délivrer. Le corps de pompe 24 porte un piston 32 rapporté sur le cylindre intérieur 28. Par ailleurs, la partie fixe 20 de la pompe 12 comprend un collier de fixation 34, permettant de sertir la pompe 12 sur le réservoir 16. Alternativement, la pompe 12 peut également être montée sur le réservoir 16 par vissage ou par encliquetage (aussi appelé « snap-on ») tel que décrit plus loin en référence aux figures 6 et 7.

Le piston 32 comprend un support 36 et une membrane 38 formant valve anti-retour plaquée sur le support 36.

Dans cet exemple, le support 36 a une forme globalement tubulaire et est monté fixe sur le cylindre intérieur 28. Il est traversé par un canal d'alimentation 40, agencé dans le prolongement du tube plongeur 30, et débouchant sur un orifice d'alimentation prévu sur l'extrémité supérieure du support 36. De façon alternative, le support 36 peut être venu de moulage avec le corps de pompe 24, et être constitué par le cylindre intérieur 28, le canal d'alimentation 40 pouvant alors être constitué par tout ou une partie du cylindre intérieur 28. L'extrémité supérieure du support 36 est coiffée par la membrane 38 et définit un siège d'appui 42 de cette membrane 38. La membrane 38 est plaquée par déformation élastique contre le siège d'appui 42. Telle que visible sur la figure 3, la membrane 38 comporte une jupe cylindrique 44 portant des moyens d'étanchéité 46.

La partie mobile 22 de la pompe 12 comprend un premier cylindre 48, monté coulissant à l'intérieur du corps de pompe 24, et délimitant, avec le piston 32, plus précisément avec la membrane 38, la chambre de dosage 50. En d'autres termes, le piston 32 est monté coulissant dans le premier cylindre 48, donc dans la chambre de dosage 50. Le coulissement étanche du piston 32, donc de la membrane 38, dans la chambre de dosage 50 est assuré par les moyens d'étanchéité 46 portés par la jupe cylindrique 44 de la membrane 38 en contact avec la paroi de la chambre de dosage 50.

La chambre de dosage 50 définit ainsi un volume de dosage, correspondant à la différence entre le volume de la chambre de dosage 50 lorsque le piston 32 est en position haute dans la chambre de dosage 50, c'est-à-dire lorsque la pompe 12 est en position activée de repos, et le volume de la chambre de dosage 50 lorsque le piston 32 est en position basse dans la chambre de dosage 50, c'est-à-dire lorsque la pompe 12 est en position de repos. Ce volume de dosage détermine la dose de produit délivré à chaque activation du dispositif 10. Le volume de la chambre de dosage 50 peut être avantageusement adapté en modifiant la longueur du support 36, tous les autres éléments du dispositif 10 restant inchangés. Le volume de la chambre de dosage 50 peut également être adapté en modifiant le diamètre du piston 32 et de la chambre de dosage 50 et/ou la course de la partie mobile 22 par rapport à la partie fixe 20.

La partie mobile 22 comporte par ailleurs un second cylindre 52, réalisé d'une seule pièce avec le premier cylindre 48. Bien sûr, les premier et second cylindres 48 et 52 peuvent être réalisés en plusieurs pièces. Un pointeau 54 est monté coulissant à l'intérieur de ce second cylindre 52, entre une position de repos et une position activée, sous l'action de premiers moyens de rappel 56, composés, dans ce mode de réalisation, d'un ressort en compression. Le pointeau 54 est configuré pour pouvoir obturer, en position de repos de la pompe 12, un orifice de sortie 58 ménagé sur l'extrémité inférieure du second cylindre 52, et comprend une extrémité 60 (visible sur la figure 3), faisant légèrement saillie à l'intérieur de la chambre de dosage 50 lorsque la pompe 12 est en position de repos. Cette extrémité 60 est configurée pour appuyer sur la membrane 38 lorsque la partie mobile 22 est en position activée, de façon à garantir l'ouverture de l'orifice de sortie 58, notamment en phase d'amorçage du dispositif 10 afin de permettre d'expulser l'air contenu dans la chambre de dosage 50 avant la première utilisation du dispositif 10.

La partie mobile 22 comporte par ailleurs un élément intermédiaire 62, monté fixe par rapport aux premier 48 et second 52 cylindres. C'est généralement sur l'élément intermédiaire 62 qu'est monté l'embout de distribution 14 du dispositif 10.

L'embout de distribution 14 comporte une enveloppe 64 comprenant un conduit 66 débouchant dans sa partie supérieure sur un orifice de pulvérisation 68 de faible diamètre ainsi que des appui-doigts 70 qui permettent à l'utilisateur d'actionner le dispositif 10.

Le cylindre extérieur 26 du corps de pompe 24 comporte un orifice de passage d'air 72 depuis l'extérieur vers l'intérieur du réservoir. Cet orifice de passage d'air 72 est obturé par un organe perméable à l'air 74 réalisé en matériau polymère ce matériau étant non poreux, par exemple en silicone.

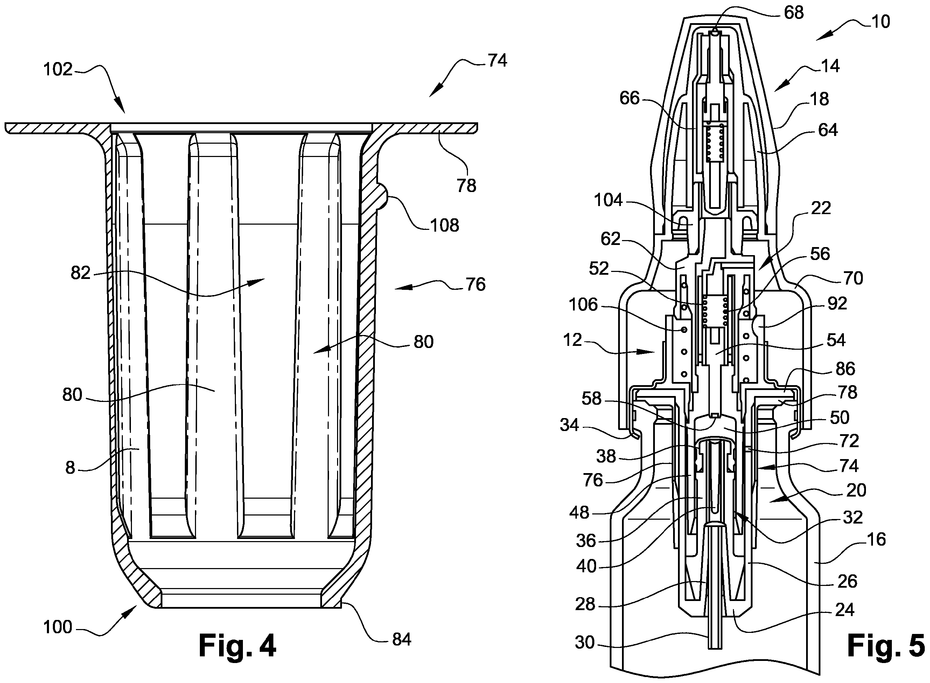

L'organe perméable à l'air 74 est également représenté sur la figure 4. L'organe perméable à l'air 74 est élastiquement déformable et présente un axe central qui est confondu, lorsque l'organe perméable à l'air 74 est monté dans le dispositif 10, avec l'axe central du corps de pompe 24. Il comporte une jupe cylindrique ayant une forme générale de manchon 76 formant une paroi de passage d'air et une portion radiale 78 formant en partie joint d'étanchéité entre le réservoir 16 et le corps de pompe 24. La portion radiale 78 est disposée à une extrémité supérieure 102 de l'organe perméable à l'air 74. Le manchon 76 comporte des nervures de renfort 80 disposées sur une surface intérieure 82 du manchon 76 et ménageant un espace libre entre la surface intérieure 82 du manchon 76 et le corps de pompe 24, dans lequel l'air peut circuler ou être stocké temporairement avant de passer par diffusion, à travers de l'organe perméable à l'air 74, dans le réservoir 16. Ces nervures de renfort 80 permettent de rigidifier l'organe perméable à l'air 74, ce qui facilite le moulage et l'assemblage de l'organe perméable à l'air 74 tout en garantissant un équilibrage relativement rapide des pressions de chaque côté de l'organe perméable à l'air 74 grâce notamment à l'épaisseur réduite de la paroi de passage d'air. Ainsi, on minimise les risques de formation de bulles d'air dans la chambre de dosage 50. Par exemple, l'épaisseur du manchon 76 peut être de 0,2 mm, et comporter sur sa surface intérieure 82 une pluralité de nervures de renfort 80 ayant une épaisseur de 0,6 mm, les nervures de renfort 80 représentant, par exemple, 60% de la surface du manchon 76. Le manchon 76 porte trois grains de riz 108, dont l'un est visible sur la figure 4, préservant avec la portion radiale 78 un espace dont la fonction est décrite plus loin.

A une extrémité inférieure 100, opposée à l'extrémité supérieure 102, l'organe perméable à l'air 74 comporte un bord libre 84 élastiquement déformable.

L'assemblage de l'organe perméable à l'air 74 sur le corps de pompe 24 est simple et est adapté à la production à l'échelle industrielle. On déforme élastiquement l'organe perméable à l'air 74 pour l'enfiler sur le corps de pompe 24. Lorsque la portion radiale 78 de l'organe perméable à l'air 74 vient en butée contre une collerette radiale 86 de fixation du corps de pompe 24 sur le réservoir 16, l'organe perméable à l'air 74 est correctement positionné autour du corps de pompe 24.

L'organe perméable à l'air 74 est fixé par auto-serrage mécanique élastique autour du corps de pompe 24. Le bord libre 84 assure l'étanchéité entre l'organe perméable à l'air 74 et le corps de pompe 24 de sorte que l'air entrant dans le dispositif 10 ne peut pas pénétrer dans le réservoir 16 sans diffuser à travers la paroi de l'organe perméable à l'air 74. Les nervures de renfort 80 permettent également un assemblage de l'organe perméable à l'air 74 sur le corps de pompe 24, par déformation élastique de l'organe perméable à l'air 74 et auto-serrage mécanique élastique des nervures de renfort 80 autour du corps de pompe 24.

Dans le premier mode de réalisation des figures 1 à 4, le dispositif 10 comprend également un joint 88 interposé entre la portion radiale 78 de l'organe perméable à l'air 74 et le réservoir 16. Au montage, ce joint 88 peut être enfilé sur le manchon 76 de l'organe perméable à l'air 74, jusqu'au-delà des grains de riz 108 qui le retiennent en place. Comme détaillé sur la figure 3, ce joint 88 forme un bourrelet 90 enveloppant radialement la portion radiale 78 de l'organe perméable à l'air 74 et formant en partie joint d'étanchéité entre le réservoir 16 et le corps de pompe 24. A cet effet, le diamètre extérieur de la portion radiale 78 de l'organe perméable à l'air 74 est inférieure à celui du joint 88 qui est sensiblement égal à celui du col du réservoir 16. Le joint 88 pourrait être interposé entre la portion radiale 78 de l'organe perméable à l'air 74 et la collerette radiale 86 du corps de pompe 24.

Par ailleurs, le dispositif 10 comporte une bague de centrage 92 et de butée haute du premier cylindre 48 de la partie mobile 22. Cette bague de centrage 92 comprend une jupe cylindrique 94 emboîtée dans le corps de pompe 24 et présentant une surface 96 venant en serrage mécanique étanche avec le premier cylindre 48 lorsque la pompe 12 est en position de repos.

A chaque utilisation du dispositif 10, l'utilisateur active la pompe 12 en appuyant sur les appui-doigts 70, le produit contenu dans la chambre de dosage 50 est expulsé hors de la chambre de dosage 50 par l'orifice de sortie 58. Il ne peut pas sortir de la chambre de dosage 50 par le canal d'alimentation 40 parce que la pression exercée par le liquide sur la membrane 38 la plaque contre le support 36 et empêche le liquide de retourner dans le réservoir 16.

Lorsque la pompe 12 est en position activée, la surface 96 n'est plus en contact étanche avec le premier cylindre 48 de la partie mobile 22 et un passage d'air 98 depuis l'extérieur vers l'intérieur du dispositif 10 qui traverse l'orifice de passage d'air 72 est ouvert, comme représenté par les flèches 98 sur la figure 2. Toutefois, ce passage d'air 98 est obturé de façon non-étanche à l'air par l'organe perméable à l'air 74.

Ensuite, lorsque l'utilisateur relâche la pression sur les appui-doigts 70, la partie mobile 22 de la pompe 12 est rappelée en position de repos, sous l'action de deuxièmes moyens de rappel 106 composés, dans ce mode de réalisation d'un ressort en compression, et la surface 96 reprend la position dans laquelle elle est en serrage mécanique étanche avec le premier cylindre 48.

Ainsi, à chaque l'activation de la pompe 12, il se produit une compensation partielle de la dépression créée à l'intérieur du réservoir 16 par reprise d'air non contaminé dans le réservoir 16.

Par exemple, s'il l'on considère un dispositif 10 avec un réservoir 16 rempli de 10 mL (soit rempli à 70 % en volume de la contenance du réservoir 16) d'un produit et muni d'un organe perméable à l'air 74 comprenant du silicone ayant une perméabilité à l'oxygène de 1,4.10⁻¹³ mol.m⁻¹.Pa⁻¹.s⁻¹ (mol par mètre par Pascal et par seconde) et une paroi d'échange de 340 mm² d'épaisseur 0,6 mm et 240 mm² d'épaisseur 0,2 mm, la délivrance d'une posologie de 3 fois 4 sprays par jour sur une période de 10 heures (soit les 4 sprays espacés de 5 heures) sous pression atmosphérique et à 25°C, c'est-à-dire 3*4*50 = 600 µL de liquide par jour, il faut environ 17 jours consécutifs pour vidanger complètement le réservoir 16. Ainsi la dépression minimale, en fin de vidange du réservoir 16, sera de 329 mbar contre 700 mbar pour un dispositif sans reprise d'air.

Ainsi, bien que le passage d'air 98 ne soit pas ouvert en permanence, la reprise d'air non contaminé dans le réservoir 16 est suffisante pour éviter la formation de bulles dans la chambre de dosage 50 et éviter que la pompe 12 ne se bloque avant que le réservoir 16 puisse être complètement vidé. On peut donc délivrer des doses précises et reproductibles de produit pendant toute l'utilisation du dispositif 10.

Comme lors du stockage du dispositif 10 avant utilisation, le passage d'air 98 est fermé, le risque de perte de produit par évaporation à travers l'organe perméable à l'air 74 est donc réduit. La dose de principe actif délivré à chaque activation de la pompe 12 n'est donc pas altérée.

Dans ce qui suit, les éléments communs aux différents modes de réalisation sont identifiés par les mêmes références numériques.

Le deuxième mode de réalisation du dispositif 10 représenté sur la figure 5 diffère du premier mode de réalisation en ce que la portion radiale 78 de l'organe perméable à l'air 74 forme seule le joint d'étanchéité entre le réservoir 16 et le corps de pompe 24. On évite ainsi de positionner une pièce supplémentaire dans le dispositif 10.

Le troisième mode de réalisation du dispositif 10 représenté sur les figures 6 et 7 diffère du premier mode de réalisation en ce que la pompe 12 et l'embout de distribution 14 sont montés sur le réservoir 16 par encliquetage (aussi appelé « snap-on »). En outre, la figure 6 présente un support 36 différent. On constate qu'en fonction de la taille et de la forme des supports 36, le volume de la chambre de dosage 50 peut être adapté sans avoir à modifier les autres éléments de la pompe 12.

Le quatrième mode de réalisation du dispositif 10 représenté sur la figure 8 diffère du premier mode de réalisation en ce que l'organe perméable à l'air 74 entoure au moins partiellement la partie fixe 20 et la partie mobile 22 de la pompe 12.

L'organe perméable à l'air 74 a une forme de manchon 76 formant la paroi de passage d'air. Le manchon 76 est muni d'une pluralité de reliefs, dans le cas présent en accordéon, ce qui permet d'augmenter la surface d'échange d'air entre l'intérieur et l'extérieur du réservoir 16 sans pour autant augmenter considérablement l'encombrement de l'organe perméable à l'air 74. En outre, les reliefs en accordéon permettent une déformation plus aisée de l'organe perméable à l'air 74 lors de l'activation de la pompe 12, c'est-à-dire lors du déplacement de la partie mobile 22 par rapport à la partie fixe 20.

Dans ce mode de réalisation, l'organe perméable à l'air 74 est fixé sur le dispositif 10 d'une part, à son extrémité inférieure 100 par auto-serrage mécanique élastique autour du collier de fixation 34 et de la bague de centrage 92 et d'autre part, à son extrémité supérieure 102 par pincement entre deux éléments de l'embout de distribution 14, dans ce mode de réalisation, entre l'enveloppe 64 et un fût de fixation 104 de l'embout de distribution 14 sur l'élément intermédiaire 62 de la partie mobile 22. On comprend que bien que n'étant pas fixé directement sur la partie mobile 22 de la pompe 12, l'organe perméable à l'air 74 entoure partiellement la partie mobile 22.

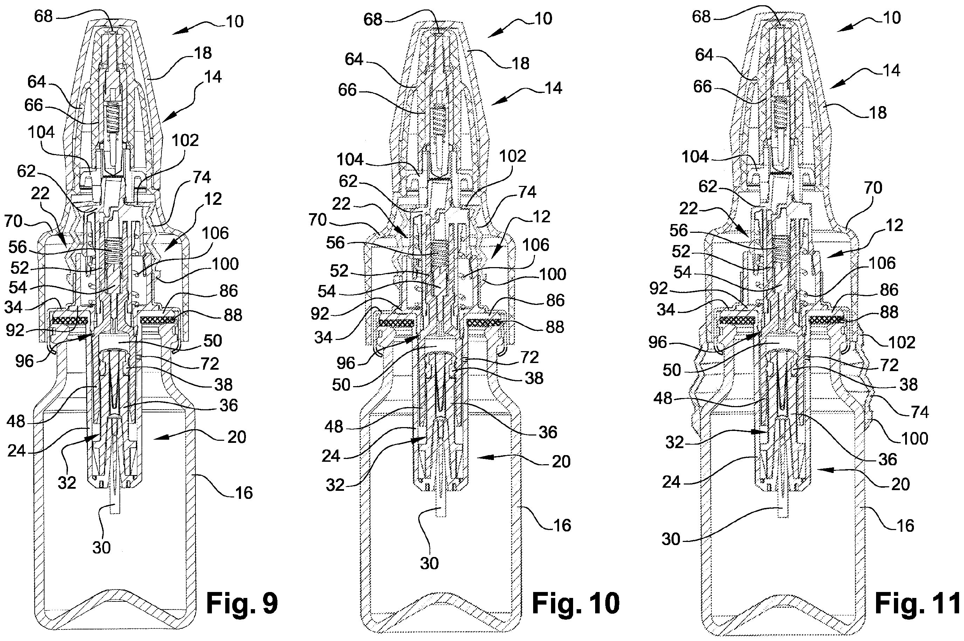

Le cinquième mode de réalisation du dispositif 10 représenté sur la figure 9 diffère du quatrième mode de réalisation en ce que l'extrémité supérieure 102 de l'organe perméable à l'air 74 est fixée par pincement entre l'élément intermédiaire 62 et le fût de fixation 104.

Le mode de réalisation non revendiqué du dispositif 10 représenté sur la figure 10 diffère du quatrième mode de réalisation en ce que l'extrémité supérieure 102 de l'organe perméable à l'air 74 est fixé par auto-serrage mécanique élastique autour de l'élément intermédiaire 62.

Le mode de réalisation non revendiqué du dispositif 10 représenté sur la figure 11 diffère des modes de réalisation précédents en ce que l'organe perméable à l'air 74 est fixé sur le dispositif 10 d'une part, à son extrémité inférieure 100 par auto-serrage mécanique élastique sur le réservoir 16 et d'autre part, à son extrémité supérieure 102 par auto-serrage mécanique élastique autour de l'enveloppe 64.

L'invention n'est pas limitée aux modes de réalisation présentés et d'autres modes de réalisation apparaîtront clairement à l'homme du métier. Il est notamment possible de modifier la membrane 38 formant valve anti-retour ou en combinant les différents modes de réalisation de l'organe perméable à l'air 74 avec différents moyens de fixation de la pompe 12 sur le réservoir 16.

## Revendications

1. Dispositif (10) pour la délivrance d'un produit par pulvérisation, comportant :
- un réservoir (16) pour le stockage du liquide à distribuer, et
- une pompe (12) comportant un corps de pompe (24) monté fixe sur le réservoir (16), le corps de pompe (24) comportant un orifice de passage d'air (72) depuis l'extérieur vers l'intérieur du réservoir (16),
- le dispositif (10) comportant un passage d'air (98) depuis l'extérieur vers l'intérieur du réservoir (16), traversant l'orifice de passage d'air (72), le passage d'air (98) étant obturé par un organe réalisé en matériau polymère perméable à l'air, ce matériau étant non poreux, l'organe étant appelé organe perméable à l'air (74)
**caractérisé en ce que** l'organe perméable à l'air (74) est fixé par pincement entre deux éléments du dispositif (10) rapportés l'un sur l'autre.

2. Dispositif (10) selon la revendication précédente, dans lequel le corps de pompe (24) et l'organe perméable à l'air (74) ont chacun un axe central, les deux axes sont confondus et l'organe perméable à l'air (74) a de préférence une forme générale de manchon (76).

3. Dispositif (10) selon la revendication précédente, dans lequel l'organe perméable à l'air (74) est élastiquement déformable et fixé par auto-serrage mécanique élastique autour d'au moins un élément du dispositif (10).

4. Dispositif (10) selon l'une quelconque des revendications précédentes, dans lequel l'organe perméable à l'air (74) comporte une paroi dite de passage d'air, munie d'une pluralité de reliefs, notamment une paroi ondulée, à section sinusoïdale, en accordéon, en créneaux ou en zig-zag.

5. Dispositif (10) selon l'une quelconque des revendications précédentes, dans lequel la pompe (12) peut prendre une position de repos et une position activée, le passage d'air (98) étant fermé lorsque la pompe (12) est en position de repos.

6. Dispositif (10) selon l'une quelconque des revendications précédentes, dans lequel l'organe perméable à l'air (74) est rapporté autour du corps de pompe (24).

7. Dispositif (10) selon l'une quelconque des revendications précédentes, dans lequel l'organe perméable à l'air (74) comporte des nervures de renfort (80) ménageant un espace libre entre une surface intérieure (82) de l'organe perméable à l'air (74) et le corps de pompe (24).

8. Dispositif (10) selon les revendications 6 ou 7, dans lequel l'organe perméable à l'air (74) comprend en outre, une portion radiale (78) formant au moins en partie joint d'étanchéité entre le réservoir (16) et le corps de pompe (24), la portion radiale (78) étant fixée, par exemple par pincement, entre le réservoir (16) et le corps de pompe (24).

9. Dispositif (10) selon la revendication précédente, dans lequel la portion radiale (78) de l'organe perméable à l'air (74) est séparée du réservoir (16) ou du corps de pompe (24) par un joint (88) formant un bourrelet (90) enveloppant radialement la portion radiale (78) de l'organe perméable à l'air (74) et formant au moins en partie joint d'étanchéité entre le réservoir (16) et le corps de pompe (24).

## Patentansprüche

1. Vorrichtung (10) zur Ausgabe eines Produkts durch Zerstäuben, mit:
- einem Behälter (16) zum Bevorraten der auszugebenden Flüssigkeit und
- einer Pumpe (12), die einen Pumpenkörper (24) aufweist, der an dem Behälter (16) fest montiert ist, wobei der Pumpenkörper (24) eine Öffnung für den Durchlass von Luft (72) von außerhalb nach innerhalb des Behälters (16) aufweist,
wobei die Vorrichtung (10) einen Durchlass von Luft (98) von außerhalb nach innerhalb des Behälters (16) aufweist, der die Luftdurchlassöffnung (72) durchquert, wobei der Luftdurchlass (98) durch ein Organ verschlossen ist, das aus luftdurchlässigem Polymermaterial realisiert ist, wobei dieses Material nicht porös ist, wobei das Organ luftdurchlässiges Organ (74) genannt wird,
**dadurch gekennzeichnet, dass** das luftdurchlässige Organ (74) durch Einklemmen zwischen zwei Elementen der Vorrichtung (10) befestigt ist, die aneinander angebracht sind.

2. Vorrichtung (10) nach dem vorhergehenden Anspruch, wobei der Pumpenkörper (24) und das luftdurchlässige Organ (74) jeweils eine mittlere Achse haben, die zwei Achsen zusammenfallen und das luftdurchlässige Organ (74) vorzugsweise eine allgemeine Form einer Hülse (76) hat.

3. Vorrichtung (10) nach dem vorhergehenden Anspruch, wobei das luftdurchlässige Organ (74) elastisch verformbar und durch elastisches mechanisches Selbstklemmen um mindestens ein Element der Vorrichtung (10) herum befestigt ist.

4. Vorrichtung (10) nach einem der vorhergehenden Ansprüche, wobei das luftdurchlässige Organ (74) eine so genannte Luftdurchlasswand aufweist, die mit einer Mehrzahl von Erhöhungen versehen ist, insbesondere eine gewellte Wand, mit sinusförmigem Querschnitt, ziehharmonikaförmig, zinnenförmig oder zickzackförmig.

5. Vorrichtung (10) nach einem der vorhergehenden Ansprüche, wobei die Pumpe (12) eine Ruhestellung und eine betätigte Stellung einnehmen kann, wobei der Luftdurchlass (98) geschlossen ist, wenn die Pumpe (12) in Ruhestellung ist.

6. Vorrichtung (10) nach einem der vorhergehenden Ansprüche, wobei das luftdurchlässige Organ (74) um den Pumpenkörper (24) herum angebracht ist.

7. Vorrichtung (10) nach einem der vorhergehenden Ansprüche, wobei das luftdurchlässige Organ (74) Verstärkungsrippen (80) aufweist, die einen freien Raum zwischen einer inneren Fläche (82) des luftdurchlässigen Organs (74) und dem Pumpenkörper (24) vorsehen.

8. Vorrichtung (10) nach Anspruch 6 oder 7, wobei, das luftdurchlässige Organ (74) ferner einen radialen Teil (78) umfasst, der zumindest teilweise ein Dichtungselement zwischen dem Behälter (16) und dem Pumpenkörper (24) bildet, wobei der radiale Teil (78), zum Beispiel, durch Einklemmen, zwischen dem Behälter (16) und dem Pumpenkörper (24) befestigt ist.

9. Vorrichtung (10) nach dem vorhergehenden Anspruch, wobei der radiale Teil (78) des luftdurchlässigen Organs (74) von dem Behälter (16) oder von dem Pumpenkörper (24) durch eine Stoßstelle (88) getrennt ist, die einen Wulst (90) bildet, der den radialen Teil (78) des luftdurchlässigen Organs (74) radial umgibt und zumindest teilweise ein Dichtungselement zwischen dem Behälter (16) und dem Pumpenkörper (24) bildet.

## Claims

1. Device (10) for delivering a product by spraying, comprising:
- a reservoir (16) for storing the liquid that is to be dispensed, and
- a pump (12) comprising a pump body (24) mounted fixedly on the reservoir (16), the pump body (24) comprising an air passage orifice (72) for the passage of air from outside to inside the reservoir (16),
- the device (10) comprising an air passage (98) from outside to inside the reservoir (16), passing through the air passage orifice (72), the air passage (98) being closed off by a member made from an air-permeable polymer material, this material being nonporous, the member being referred to as an air-permeable member (74),
**characterized in that** the air-permeable member (74) is fixed by clamping between two elements of the device (10) which are joined together.

2. Device (10) according to the preceding claim, in which the pump body (24) and the air-permeable member (74) each have a central axis, the two axes are coincident and the air-permeable member (74) preferably has the overall shape of a sleeve tube (76).

3. Device (10) according to the preceding claim, in which the air-permeable member (74) is elastically deformable and is fixed around at least one element of the device (10) by elastic mechanical self-clamping.

4. Device (10) according to any one of the preceding claims, in which the air-permeable member (74) comprises a wall referred to as an air-passage wall, provided with a plurality of reliefs, notably an undulating wall, the cross section of which is sinusoidal, concertinaed, crenelated or zigzag.

5. Device (10) according to any one of the preceding claims, in which the pump (12) can take a rest position and an activated position, the air passage (98) being closed when the pump (12) is in the rest position.

6. Device (10) according to any one of the preceding claims, in which the air-permeable member (74) is attached around the pump body (24).

7. Device (10) according to any one of the preceding claims, in which the air-permeable member (74) comprises reinforcing ribs (80) creating an empty space between an interior surface (82) of the air-permeable member (74) and the pump body (24).

8. Device (10) according to Claims 6 or 7, in which the air-permeable member (74) further comprises a radial portion (78) at least partially forming a seal between the reservoir (16) and the pump body (24), the radial portion (78) being fixed, for example by clamping, between the reservoir (16) and the pump body (24).

9. Device (10) according to the preceding claim, in which the radial portion (78) of the air-permeable member (74) is separated from the reservoir (16) or from the pump body (24) by a seal (88) forming a bulge (90) radially enveloping the radial portion (78) of the air-permeable member (74) and at least partially forming a seal between the reservoir (16) and the pump body (24).
